# EUROPEAN PATENT APPLICATION

(11) **EP 3 912 587 A1**
(43) Date of publication of application: **24.11.2021**
(21) Application number: 21174840.5
(22) Date of filing: 19.05.2021
(51) Int. Cl.: A61B 34/20

(54) **STABILIZATION SYSTEM FOR NAVIGATION CAMERA IN COMPUTER-ASSISTED SURGERY**

(30) Priority: 19.05.2020 US 202063026847 P
(71) Applicant: Orthosoft ULC, Montréal, QC H3C 2N6 (CA)
(72) Inventor: SORIANO, Mickael, 34790 Grabels (FR)
(74) Representative: Taor, Simon Edward William

(57) **Abstract**

A system for tracking at least one tool and/or at least one bone during computer-assisted surgery includes a processing unit; and a non-transitory computer-readable memory communicatively coupled to the processing unit and comprising computer-readable program instructions executable by the processing unit for: tracking at least one tool and/or at least one bone with at least one image-capture device, with the image-capture device being at a first orientation during a surgical procedure, detecting a change in orientation of the at least one image-capture device from the first orientation, quantifying the change in orientation of the at least one image-capture device from the first orientation, and tracking of the at least one tool and/or of the at least one bone with the at least one image-capture device as a function of the quantifying of the change in orientation of the image-capture device from the first orientation.

## Description

### CROSS-REFERENCE TO RELATED APPLICATION

The present application claims the benefit of United States Patent Application No. 63/026,847, entitled "STABILIZATION SYSTEM FOR NAVIGATION CAMERA IN COMPUTER-ASSISTED SURGERY" filed on May 19, 2020, the contents of which are incorporated herein by reference.

### TECHNICAL FIELD

The present application relates to navigation in computer-assisted surgery, such as orthopedic surgery.

### BACKGROUND

Navigation technologies are commonly used in computer-assisted surgery. Navigation technologies may involve the use of cameras or like imaging device that may track objects such as patient tissues and tools during a surgical or dental procedure. The objects may consequently be tracked in a global referential system for the relative positions and orientations of the objects to be calculable in real time.

As such procedures often imply high levels of accuracy and precision, various calibration steps may be required before and during the procedures. The camera or like imaging device is at the core of the global referential system, due to its fixed position in numerous set-ups. However, it may occur that medical or dental personnel come into contact with the camera, and this may have the effect of de-calibrating the camera relative to the global referential system. The procedure may consequently be halted for the various objects to be recalibrated. This may for example lengthen the duration of surgery.

### SUMMARY

In accordance with a first aspect of the present disclosure, there is provided a system for tracking at least one tool and/or at least one bone during computer-assisted surgery, comprising: a processing unit; and a non-transitory computer-readable memory communicatively coupled to the processing unit and comprising computer-readable program instructions executable by the processing unit for: tracking at least one tool and/or at least one bone with at least one image-capture device, with the image-capture device being at a first orientation during a surgical procedure, detecting a change in orientation of the at least one image-capture device from the first orientation, quantifying the change in orientation of the at least one image-capture device from the first orientation, and tracking of the at least one tool and/or of the at least one bone with the at least one image-capture device as a function of the quantifying of the change in orientation of the image-capture device from the first orientation.

Further in accordance with the first aspect, for example, tracking as a function of the quantifying of the change in orientation of the at least one image-capture device from the first orientation includes automating a return of the image-capture device to the first orientation.

Still further in accordance with the first aspect, for example, automating the return of the at least one image-capture device to the first orientation includes actuating a movement of the at least one image-capture device in one or two rotational degrees of freedom.

Still further in accordance with the first aspect, for example, automating the return of the at least one image-capture device to the first orientation is performed automatically after the detecting and the quantifying.

Still further in accordance with the first aspect, for example, tracking as a function of the quantifying of the change in orientation of the image-capture device from the first orientation includes factoring in an adjustment of a point of view of the at least one image-capture device.

Still further in accordance with the first aspect, for example, a user is alerted of the detecting of the change in orientation of the at least one image-capture device.

Still further in accordance with the first aspect, for example, the user is required to quantify and/or validate the change.

Still further in accordance with the first aspect, for example, the tracking of the at least one tool and/or at least one bone is paused between the detecting and the qualifying, and resuming the tracking after the quantifying.

Still further in accordance with the first aspect, for example, detecting the change in orientation of the at least one image-capture device from the first orientation includes detecting the change in orientation when the at least one image-capture device is coupled to a stationary structure.

Still further in accordance with the first aspect, for example, detecting the change in orientation includes continuously monitoring the orientation of the at least one image-capture device.

Still further in accordance with the first aspect, for example, the tracking of the at least one tool and/or of the at least one bone is output.

Still further in accordance with the first aspect, for example, outputting the tracking of the at least one tool and/or of the at least one bone includes outputting the tracking graphically on a graphic-user interface.

Still further in accordance with the first aspect, for example, tracking at least one tool and/or at least one bone includes tracking the at least one tool relative to the at least one bone.

Still further in accordance with the first aspect, for example, tracking the at least one tool includes tracking the at least one tool as moved by a surgical robot, and further including blocking movement of the surgical robot when detecting the change in orientation.

In accordance with a second aspect of the present disclosure, there is provided a stabilization system for a navigation image-capture device in computer-assisted surgery comprising: an assembly providing at least two rotational degrees of freedom (DOF), the assembly configured to interface a navigation image-capture device to a support structure or mechanism; and at least one sensor coupled to the assembly to output data indicative of a change of orientation for the at least two rotational DOFs of the navigation image-capture device.

Further in accordance with the second aspect, for example, the assembly includes a universal joint.

Still further in accordance with the second aspect, for example, the at least one sensor includes at least one encoder coupled to the rotational DOFs.

Still further in accordance with the second aspect, for example, at least one redundant sensor produces data relative to the change of orientation for the at least two rotational DOFs of the navigation image-capture device.

Still further in accordance with the second aspect, for example, the at least one redundant sensor includes at least one inertial sensor.

Still further in accordance with the second aspect, for example, the stabilization system may further include: a processing unit; and a non-transitory computer-readable memory communicatively coupled to the processing unit and comprising computer-readable program instructions executable by the processing unit for: tracking at least one tool and/or at least one bone with the navigation image-capture device at a first orientation during a surgical procedure, detecting the change in orientation of the navigation image-capture device from the first orientation, quantifying the change in orientation of the navigation image-capture device from the first orientation, and tracking of the at least one tool and/or of the at least one bone with the navigation image-capture device as a function of the quantifying of the change in orientation of the image-capture device from the first orientation.

Still further in accordance with the second aspect, for example, tracking as a function of the quantifying of the change in orientation of the navigation image-capture device from the first orientation includes factoring in an adjustment of a point of view of the navigation image-capture device.

Still further in accordance with the second aspect, for example, movements of a surgical robot are blocked when the change in orientation is detected.

Still further in accordance with the second aspect, for example, actuators are in the assembly to modify the orientation of the assembly in the at least two rotational DOFs.

Still further in accordance with the second aspect, for example, the stabilization system may further include a processing unit; and a non-transitory computer-readable memory communicatively coupled to the processing unit and comprising computer-readable program instructions executable by the processing unit for: tracking at least one tool and/or at least one bone with the navigation image-capture device at a first orientation during a surgical procedure, detecting the change in orientation of the navigation image-capture device from the first orientation, quantifying the change in orientation of the navigation image-capture device from the first orientation, automating a return of the navigation image-capture device to the first orientation, and resuming the tracking of the at least one tool and/or of the at least one bone with the navigation image-capture device after the return of the image-capture device to the first orientation.

Still further in accordance with the second aspect, for example, automating the return of the navigation image-capture device to the first orientation includes actuating a movement of the at least one image-capture device in one or two of the rotational DOFs.

Still further in accordance with the second aspect, for example, automating the return of the navigation image-capture device to the first orientation is performed automatically after the detecting and the quantifying.

Still further in accordance with the second aspect, for example, movements of a surgical robot are blocked when the change in orientation is detected and until the return of the navigation image-capture device to the first orientation.

Still further in accordance with the second aspect, for example, a user is alerted of the detecting of the change in orientation of the navigation image-capture device.

Still further in accordance with the second aspect, for example, input from the user is required to quantify and/or validate the change.

Still further in accordance with the second aspect, for example, the tracking of the at least one tool and/or at least one bone is paused between the detecting and the qualifying, and resuming the tracking after the quantifying.

Still further in accordance with the second aspect, for example, detecting the change in orientation of the navigation image-capture device from the first orientation includes detecting the change in orientation when the navigation image-capture device is on a stationary structure.

Still further in accordance with the second aspect, for example, detecting the change in orientation includes continuously monitoring the orientation of the navigation image-capture device.

Still further in accordance with the second aspect, for example, the tracking of the at least one tool and/or of the at least one bone is output.

Still further in accordance with the second aspect, for example, outputting the tracking of the at least one tool and/or of the at least one bone includes outputting the tracking graphically on a graphic-user interface.

Still further in accordance with the second aspect, for example, tracking at least one tool and/or at least one bone includes tracking the at least one tool relative to the at least one bone.

In accordance with a third aspect of the present disclosure, there is provided a method for tracking at least one tool and/or at least one bone during computer-assisted surgery, comprising: tracking at least one tool and/or at least one bone with at least one image-capture device, with the image-capture device being at a first orientation during a surgical procedure, detecting a change in orientation of the at least one image-capture device from the first orientation, quantifying the change in orientation of the at least one image-capture device from the first orientation, and tracking of the at least one tool and/or of the at least one bone with the at least one image-capture device as a function of the quantifying of the change in orientation of the image-capture device from the first orientation.

Further in accordance with the third aspect, for example, tracking as a function of the quantifying of the change in orientation of the at least one image-capture device from the first orientation includes automating a return of the image-capture device to the first orientation.

Still further in accordance with the third aspect, for example, automating the return of the at least one image-capture device to the first orientation includes actuating a movement of the at least one image-capture device in one or two rotational degrees of freedom.

Still further in accordance with the third aspect, for example, automating the return of the at least one image-capture device to the first orientation is performed automatically after the detecting and the quantifying.

Still further in accordance with the third aspect, for example, tracking as a function of the quantifying of the change in orientation of the image-capture device from the first orientation includes factoring in an adjustment of a point of view of the at least one image-capture device.

Still further in accordance with the third aspect, for example, a user is alerted of the detecting of the change in orientation of the at least one image-capture device.

Still further in accordance with the third aspect, for example, the user is required to quantify and/or validate the change.

Still further in accordance with the third aspect, for example, the tracking of the at least one tool and/or at least one bone is paused between the detecting and the qualifying, and resuming the tracking after the quantifying.

Still further in accordance with the third aspect, for example, detecting the change in orientation of the at least one image-capture device from the first orientation includes detecting the change in orientation when the at least one image-capture device is coupled to a stationary structure.

Still further in accordance with the third aspect, for example, detecting the change in orientation includes continuously monitoring the orientation of the at least one image-capture device.

Still further in accordance with the third aspect, for example, the tracking of the at least one tool and/or of the at least one bone is output.

Still further in accordance with the third aspect, for example, outputting the tracking of the at least one tool and/or of the at least one bone includes outputting the tracking graphically on a graphic-user interface.

Still further in accordance with the third aspect, for example, tracking at least one tool and/or at least one bone includes tracking the at least one tool relative to the at least one bone.

Still further in accordance with the third aspect, for example, tracking the at least one tool includes tracking the at least one tool as moved by a surgical robot, and further including blocking movement of the surgical robot when detecting the change in orientation.

### DESCRIPTION OF THE DRAWINGS

Fig. 1 is a schematic view of a computer-assisted surgery (CAS) system with stabilization system for navigation image-capture device in accordance with the present disclosure;
Fig. 2 is an assembly view of the stabilization system for navigation image-capture device of the CAS system of Fig. 1;
Figs. 3A and 3B are assembly views of parts of a joint and link assembly of the stabilization system of Fig. 2;
Fig. 4 is a block diagram of a controller of the stabilization system of Fig. 2; and
Fig. 5 is a flowchart showing an exemplary method for tracking tools relative to a patient during computer-assisted surgery.

### DETAILED DESCRIPTION

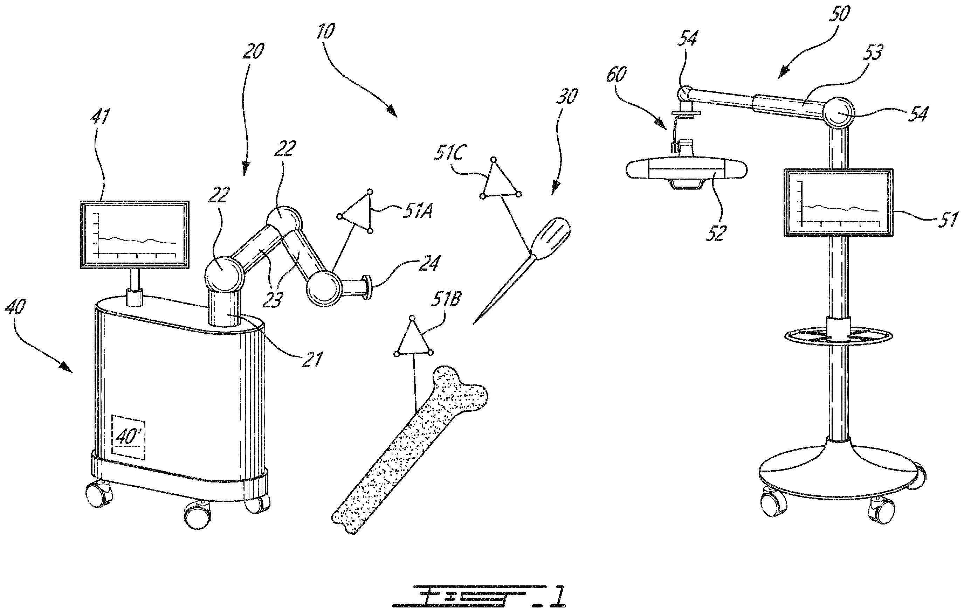

Referring to the drawings and more particularly to Fig. 1, an exemplary computer-assisted surgery (CAS) system is generally shown at 10, and is used to perform orthopedic surgery maneuvers on a patient. Orthopedic surgery is one of numerous types of patient procedures that may be assisted with the CAS system 10, and is merely given as an example as other types of procedures may benefit from computer-assisted navigation. The CAS system 10 may consequently have one or more processing units dedicated to operating a workflow of a surgical procedure. The CAS system 10 may therefore include a non-transitory computer-readable memory communicatively coupled to the one or more processing units and may comprise computer-readable program instructions executable by the one or more processing units to operate the workflow described herein. In an embodiment, the CAS system 10 drives a surgical robot 20 used autonomously, and/or as an assistive or collaborative tool for an operator (e.g., surgeon). In another embodiment, the CAS system 10 is one used without robotic assistance, and assists operator by way of surgical navigation.

The CAS system 10 is shown relative to a patient's bone, shown schematically, but only as an example. The system 10 could be used for other body parts, including non-exhaustively hip joint, spine, and shoulder bones, or in other applications, including dentistry, craniomaxillofacial, other non-orthopedic surgeries, etc.

The CAS system 10 may include the surgical robot 20, one or more surgical tools 30 such as a digitizer used in hand mode, a CAS controller as part of a station, shown as CAS controller 40, a tracking system 50, and a stabilization system 60 for navigation image-capture device:
- The surgical robot 20 is optionally present, and is one possible working end of the CAS system 10, and is used to perform bone alterations as planned by an operator and/or the CAS controller 40 and as controlled by the CAS controller 40;
- The surgical tool(s) 30 are tools that are manipulated by the operator in order to perform tasks on the patient. A digitizer is shown in Fig. 1, but other tools may be used depending on the nature of the procedure. Tools 30 may include awls, drills, impactors, saws, to name a few of the one or more tools 30 that may be used, as numerous other tools may be used. The surgical tool(s) may or may not be used depending on whether a surgical robot 20 is in the CAS system 10. It is contemplated to have both the surgical robot 20 and the surgical tool(s) 30 in the CAS system 10;
- The CAS controller 40 operates the surgical workflow. The CAS controller 40 may also control the surgical robot 20 if present. The CAS controller 40 may also guide an operator through the surgical procedure, by providing intraoperative data of position and orientation, and may therefore have the appropriate interfaces such as mouse, foot pedal, touchscreen, etc;
- The tracking system 50 may be used to track the bones and/or soft tissue of the patient, the surgical robot 20 and surgical tools 30 if present. For example, the tracking system 50 may assist in performing the calibration (or registration) of the patient bone with respect to the robot arm, for subsequent navigation in the X, Y, Z coordinate system. The tracking system 50 includes a navigation image-capture device or like imaging device, as detailed below;
- The stabilization system 60 for navigation system is tasked with restoring a calibration of a navigation image-capture device of the tracking system 50.

The CAS system 10 may be without the surgical robot 20, with the operator performing manual tasks. In such a scenario, the CAS system 10 may only have the tools 30, the CAS controller 40, the tracking system 50 and the stabilization system 60. The CAS system 10 may also have non-actuated foot support and thigh support to secure the limb.

Still referring to Fig. 1, a schematic example of the surgical robot 20 is provided. The surgical robot 20 may stand from a base 21, for instance in a fixed relation relative to the operating-room (OR) table supporting the patient. The fixed positioning of the surgical robot 20 relative to the patient is a determinative factor in the precision of the surgical procedure, whereby the base 21 may have its casters (shown) or any other support structure locked into place so as not to move. The surgical robot 20 has a plurality of joints 22 and links 23, of any appropriate form, to support a tool head 24 that interfaces with the patient. The tool head 24 may be an appropriate type of tool and may be interchangeable, as a function of the nature of the surgical procedure.

The arm of the surgical robot 20 is shown being a serial mechanism, arranged for the tool head 24 to be displaceable in a desired number of degrees of freedom (DOF). For example, the surgical robot 20 controls movements of the tool head 24. In an embodiment, the surgical robot 20 has a 6-DOF articulated arm, i.e., X, Y, Z in the coordinate system, and pitch, roll and yaw. Fewer or additional DOFs may be present. For simplicity, only a generic illustration of the joints 22 and links 23 is provided, but more joints of different types may be present to move the tool head 24 in the manner described above. The joints 22 are powered for the surgical robot 20 to move as controlled by the controller 40 in the six DOFs. Therefore, the powering of the joints 22 is such that the tool head 24 of the surgical robot 20 may execute precise movements, such as moving along a single direction in one translation DOF, or being restricted to moving along a plane, among possibilities. Such surgical robots 20 are known, for instance as described in United States Patent Application Serial nos. 11/610,728 and 12/452,142 , incorporated herein by reference.

The CAS controller 40 has a processor unit 40' (a.k.a., processing unit, such as a processor, CPU, ASIC, etc) to control movement of the surgical robot 20, if applicable. The CAS controller 40 provides computer-assisted surgery guidance to an operator, whether in the form of navigation data, model assessment, or in preoperatively planning or during the surgical procedure. The CAS controller 40 is shown being integrated into the housing in the base 21 of the surgical robot 20, but may be at other locations, for example if the CAS system 10 does not have the surgical robot 20. The CAS controller 40 may be a self-standing computer (e.g., lap top, tablet, etc) or may be integrated or supported in the base of the tracking system 50. The system 10 may comprise various types of interfaces, for the information to be provided to the operator, for instance via the graphic-user interfaces (GUIs) 41 and/or 51. The GUI 41, 51 may be monitors and/or screens including wireless portable devices (e.g., phones, tablets, augmented reality headsets), audio guidance, LED displays, among many other possibilities. If a surgical robot 20 is present, the CAS controller 40 may then drive the surgical robot 20 in performing the surgical procedure based on a pre-operative or peri-operative planning or through operator guidance intra-operatively. The CAS controller 40 runs various modules, in the form of algorithms, code, non-transitory executable instructions, etc, in order to operate the system 10 in the manner described herein. For example, the CAS controller 40 integrates non-transitory computer-readable memory communicatively coupled to the processing unit and comprising computer-readable program instructions executable by the processing unit. Stated differently, the CAS controller 40 integrates a non-transitory computer-readable medium storing instructions that, when executed by a processor, cause the processor to perform operations.

The use of the tracking system 50 may provide tracking data to perform surgical navigation. For example, the tracking system 50 may assist in performing the calibration of the patient bone with respect to the coordinate system, for subsequent navigation in the X, Y, Z coordinate system. According to an embodiment, the tracking system 50 comprises an image capturing device 52, also known as a navigation camera, that optically sees and recognizes references 51A, 51B, and 51C (e.g., retro-reflective references, optically recognizable references) - that may be part of the tools used in surgery -, so as to track the robot arm of the surgical robot 20 and/or one or more tools 30 and limbs in six DOFs, namely in position and orientation. In an embodiment featuring the surgical robot 20, the reference 51A is on the tool head 24 of the surgical robot 20 such that its tracking allows the CAS controller 40 to calculate the position and/or orientation of the tool head 24 and tool thereon. Likewise, references 51B and 51C are fixed to the patient bones, such as the tibia for reference 51 B and the femur for reference 51C. In an embodiment without the surgical robot 20, references such as reference 51A are on the navigated tools 30 (including a registration tool shown) such that their tracking allows the controller 40 to calculate the position and/or orientation of the tools and register points. Likewise, references 51B may be fixed to the patient bones. The references 51A-C attached to the patient need not be invasively anchored to the bone, as straps or like attachment means may provide sufficient grasping to prevent movement between the references 51A-C, and the bones, in spite of being attached to soft tissue. However, the references 51B and 51C could also be secured directly to the bones. Therefore, the controller 40 continuously updates the position and/or orientation of the surgical robot 20, tools 30 and patient bones in the X, Y, Z coordinate system using the data from the tracking system 50.

Although the set of references 51A, 51 B, 51C and image-capture device 52 is one of type featuring retro-reflective spheres (e.g., Navitrack® system), other image based tracking technologies may be used, such as depth cameras, 3D cameras, etc, without the need for trackers or like trackable references on objects, or with other types of trackers, such as QR codes, etc. The use of the expression "image-capture device" herein is deemed to incorporate all such imaging devices using for navigation. The image-capture device 52 is configured to generate an image, such as image data, of a field of view of the image-capture device 52. In some implementations, a controller or processor of the image-capture device 52 is configured to perform one or more operations based on or using the image data, such as one or more image processing functions. For example, the one or more operations may include object detection, object recognition, object tracking. Additionally, or alternatively, the image-capture device 52 may be configured to generate an output, such as the image data or an indicator of an operation or a result of an option. Moreover, even though the expression "image-capture device" may be used in the singular, the image-capture device 52 may include more than a single point of view, for example using triangulation as an option with two points of view.

In an embodiment, the image-capture device 52 is mounted onto a stationary structure, such as a ground stand, which may include the GUI 51, and a processor unit(optionally present, but that could be the processor unit 40 if no surgical robot 20 is present). An articulated arm featuring links 53 and joints 54 may be present, with the joints 54 being lockable or being capable of maintaining a set position and orientation, whereby the articulated arm may be regarded a stationary structure. This may allow an adjustment of the orientation of the image-capture device 52, as a line of sight may be required between the image-capture device 52 and the objects being tracked in surgery. For example, the image-capture device 52 may stand over the patient and look down on the surgical zone. The stand may be on casters or like supporting structure to fix the stand in position on the ground. It is also contemplated to use other types of structures or mechanisms to support the image-capture device 52, such a ceiling-mounted arm, a wall-mounted arm, a table mounted arm, a console-mounted arm, etc.

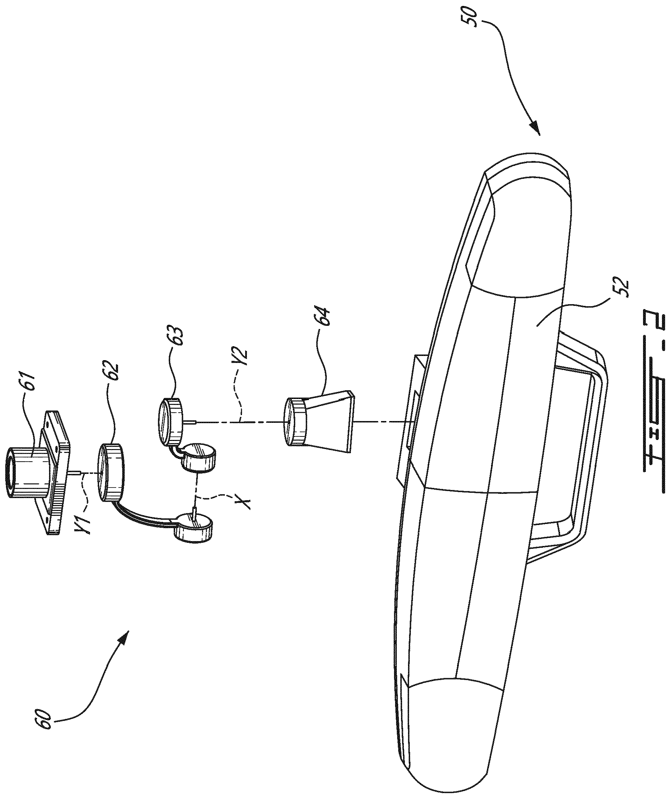

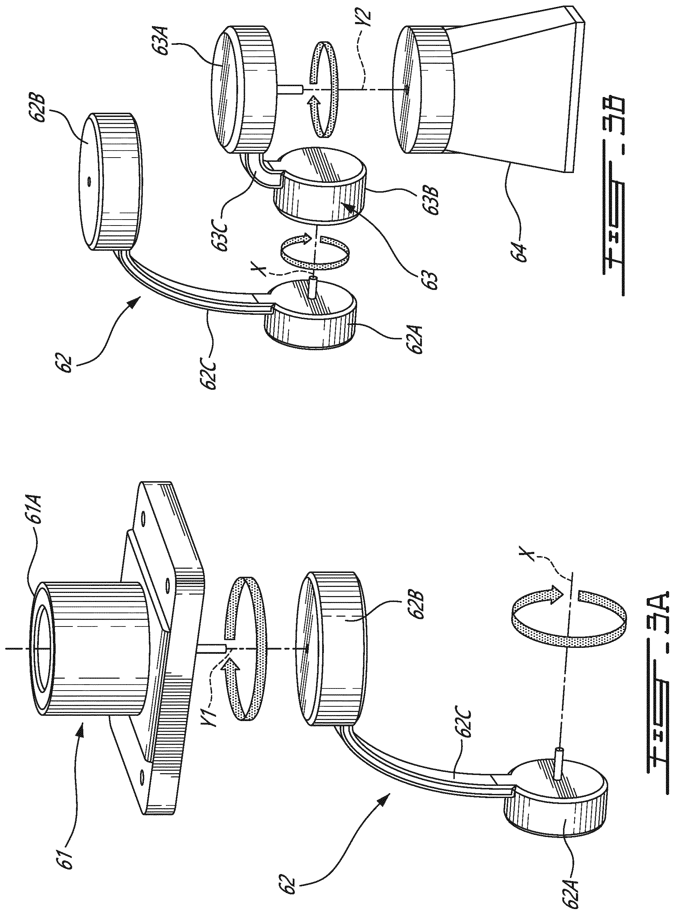

Referring to Figs. 2, 3A and 3B, the stabilization system 60 is shown in greater detail. The stabilization system 60 may have its hardware components between a link 53 or joint 54 and the image-capture device 52. The stabilization system 60 may be located elsewhere in the structure supporting the image-capture device 52. As shown in Figs. 2, 3A and 3B, the stabilization system 60 may be a set of links and rotational joint portions providing two or more rotational DOFs, for example to allow rotations about axes X, Y1 and Y2. In an embodiment, the assembly of links and rotational joints may be arranged in such a way that the axes X, Y1 and Y2 intersect one another, such that the assembly is a universal joint, with 3 rotational DOFs. The assembly of joints may be referred to as a gimbal. Only two rotational DOFs may be provided as well, such as those about axes X and Y1. The illustrated embodiment of Figs. 2, 3A and 3B is a single, multi DOF joint, but multiple joints may be present, and arranged serially. In another embodiment, the assembly is a spherical joint.

Referring to Fig.3A, the stabilization system 60 may include a connector member 61 that is mounted to an end of the articulated arm. For example, the connector member 61 is connected to a joint 53 or link 54 (Fig. 1) at a distal end of the tracking system 50. The connector member 61 may for example have a plate for connection to the joint 54 (or to a link 53), but other connection interfaces may be present. The plate is shown as being rectangular in shape, with connection bores for fasteners, as an example among numerous others. An actuator 61A may be supported by the connector member 61, and is operable to output a bi-directional rotational output, about axis Y1. The actuator 61A may for example be a brushless motor, with an encoder or other appropriate orientation sensor. Other types of actuators may be used.

A first link 62 is rotatably connected to the connector member 61, by way of connector end 62B. The first link 62 may also have an actuator, shown as actuator 62A, and operable to output a bi-directional rotational output, about axis X. The actuator 62A may also be a brushless motor, with an encoder or other appropriate orientation sensor, though other types of actuators may be used as well. The first link 62 may also have a connector end 62B that may be rotatably connected to the actuator 61A, so as to be rotated by the actuator 61A about axis Y1. For example, the connector end 62B may act as a shaft to the actuator 61A or may be connected to a shaft of the actuator 61A. The angular position of the first link 62 relative to the connector member 61 is known and tracked, by the sensor that is part of the actuator 61A, or via an inertial sensor on the first link 62, etc. Arm 62C spaces the actuator 62A apart from the connector end 62B. The arm 62C has a rigid body and has a geometrical shape that has axes X and Y1 intersect. A reverse arrangement is possible, in which the actuator for axis Y1 is in the first link 62 at 62B, while the connector member 61 would act as a shaft to the actuator at 62B.

Referring to Fig. 3B, a second link 63 is rotatably connected to the first link 62, by way of connector end 63B. The second link 63 may also have an actuator, shown as actuator 63A, and operable to output a bi-directional rotational output, about axis Y2. The actuator 63A may for example be a brushless motor, with an encoder or other appropriate orientation sensor, though other types of actuators may be used as well. The second link 63 may also have a connector end 63B that may be rotatably connected to the actuator 62A, so as to be rotated by the actuator 62A about axis X. For example, the connector end 63B may act as a shaft to the actuator 62A or may be connected to a shaft of the actuator 62A. The angular position of the second link 63 relative to the first link 62 is known and tracked by the sensor that is part of the actuator 62A, or by any other sensor. Arm 63C spaces apart and orients the actuator 63A from/relative to the connector end 63B. The arm 63C has a rigid body and has a geometrical shape that has axes X and Y2 intersect. A reverse arrangement is again possible, in which the actuator for axis X is in the second link 63 at 63B, while the first link 62 at 62A would act as a shaft to the actuator at 63B.

Still referring to Fig. 3B, a device connector 64 is rotatably connected to the second link 63. The device connector 64 may be fixed or secured to the image-capture device 52 (Fig. 1), or may be part of the image-capture device 52. The device connector 64 may be rotatably connected to the actuator 63A, so as to be rotated by the actuator 63A about axis Y2. For example, the device connector 64 may act as a shaft to the actuator 63A or may be connected to a shaft of the actuator 63A. The angular position of the device connector 64 relative to the second link 63 is known and tracked by the sensor that is part of the actuator 63A, or by any other sensor. A reverse arrangement is possible, in which the actuator for axis Y2 is in the device connector 64, while the second link 63 at 63A would act as a shaft to the actuator in the device connector 64.

Therefore, as detailed above, an orientation of the image-capture device 52 may be adjusted about axes X, Y1 and/or Y2 by the hardware assembly of the stabilization system 60, relative to the articulated arm of the camera stand, or relative to any other support structure to which the stabilization system 60 is connected. In an embodiment, the joints between the connector member 61, the first link 62, the second link 63 and the device connector 64 self block, i.e., they have sufficient friction to preserve the orientation between the connectors and/or links. Stated differently, if the image-capture device 52 is placed in desired orientation, the user may simply stop manipulating the image-capture device 52 and it will preserve its orientation. In contrast, the joints 54 of the articulated arm may lock, such that an impact on the image-capture device 52 will cause movement at the stabilization system 60 and not at the articulated arm. Stated differently, once an orientation of the image-capture device 52 is set by the stabilization system 60, the orientation is maintained. According to an embodiment, this may be done passively, i.e., without having to power the actuators 61A, 62A and/or 63A. In an embodiment, the orientation of the image-capture device 52 may however be modified by applying manual force on the image-capture device 52. It may also be possible to actuate the actuators 61A, 62A and/or 63A, to adjust the orientation of the image-capture device 52. In an embodiment, there are no actuators 61A, 62A and/or 63A, with passive rotational joints being between the end connector 61, the first link 62, the second link 63, with such rotational joints having friction to self block to preserve the orientation between the components or with such rotational joints being lockable. However, sensors are present in the joints to determine any change of orientation between the end connector 61, the first link 62, the second link 63 and the device connector 64. Stated differently, angular variations of the rotational DOFs is detected with any appropriate sensors.

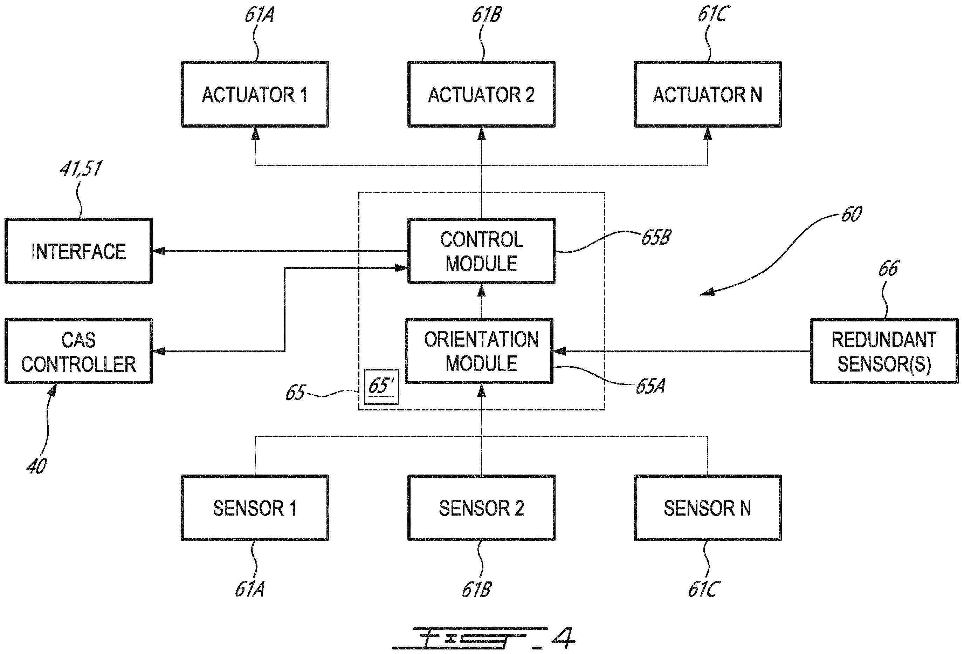

Referring to Fig. 4, the stabilization system 60 is shown featuring its hardware components, namely actuators 1 to N, and sensors 1 to N. The actuators and sensors may be paired as 61A, 61B, and 61C, as in the embodiment of Fig. 4, if both present. The stabilization system 60 has a controller 65 that receives the data from the sensors 61A-61C and drives the actuators 61A-61C (if present). The controller 65 may also communicate with the CAS controller 40 for joint operation, and may display data and/or may communicate information to an operator via the GUIs 41, 51. In an embodiment, the controller 65 has an orientation module 65A and a control module 65B. These modules may be computer-readable program instructions executable by the one or more processing units 65' of the CAS system 10 to operate the functions described hereinafter. In an embodiment, the modules 65A and 65B may be operated by the processing unit(s) 40' with the CAS controller 40.

The orientation module 65A may be tasked with determining and recording an orientation of the image-capture device 52 relative to its articulated arm, and more specifically, by monitoring the rotational DOFs of the stabilization system 60. The orientation module 65A therefore receives signals from the sensors 61A-61C, for example throughout a surgical procedure, to determine a real-time orientation of the DOFs of the stabilization system 60. Consequently, if an orientation of the image-capture device 52 changes relative to the articulated arm of the camera stand, the change in orientation may be attributed to the joint and link assembly of the stabilization system 60, as the rotational DOFs of the stabilization system 60 may be less rigid than those of the articulated arm, by design, in an example.

In an embodiment, the orientation module 65A records a first orientation of the image-capture device 52. The first orientation may be the initial orientation, i.e., prior to or at the commencement of a surgical workflow, or before or at a calibration or creation of the global referential system. The recordation may be described as setting and storing the first orientation as reference value, for example as angular values in three axes. The recordation by the orientation module 65A may in an example be triggered by a user, such as via a button or switch directly on the tracking system 50 or stabilization system 60 (e.g., the button may be on the image-capture device 52 or on the articulated arm featuring links 53 and joints 54), an interaction using the surgical workflow. The user may proceed with the trigger of recordation after having placed the image-capture device 52 correctly relative to the operative scene. In another embodiment, the recordation is automatically triggered by the surgical workflow after a given step is reached. One condition may be the stability of the image-capture device 52. As the global referential system may be a function of the initial orientation of the image-capture device 52, its recordation by the orientation module 65A may serve to replicate the initial orientation if an orientation change occurs inadvertently (e.g., by accidental contact). A redundant sensor(s) 66 may also provide an indication to the orientation module 65A of an impact or change of orientation of the image-capture device 52. For example, the redundant sensor(s) 66 may be an inertial sensor(s), such as an accelerometer unit, a gyroscope, or the like, on the image-capture device 52. In an embodiment, the redundant sensor 66 is used instead of the sensors 61A-61C to identify a change of orientation of the image-capture device 52.

The control module 65B may be used to power the actuators 61A to 61C, to return the image-capture device 52 to its initial orientation. For example, once the orientation module 65A detects and quantifies a change of orientation, about one or more of the rotational DOFs, the control module 65B may control the actuators 61A to 61C as a function of the detection and quantification, such that the image-capture device 52 may be recalibrated, i.e., returned to its initial orientation as if it had not moved. This may for instance occur automatically, or may require an approval from an operator to then be automated. The control module 65B may communicate with the CAS controller 40 to halt the workflow until the image-capture device 52 has been recalibrated, request permission to recalibrate, and/or indicate that the image-capture device 52 has been recalibrated, etc. In another embodiment, the control module 65B may receive the quantification of angular variation from the orientation module 65A, and may adjust tracking data as a function of the adjusted orientation of the image-capture device 52. Stated differently, using the geometrical data between the image-capture device 52 and the joint and link assembly of the stabilization system 60, and the quantified angular variation from the initial orientation to the uncalibrated orientation, the control module 65B, for instance jointly with the CAS controller 40, may adjust the position and orientation of the objects from the tracking data from the tracking system 50. Stated differently, the control module 65B may take into account a new point of view of the image-capture device 52 (e.g., the two POVs if more than one) in the triangulation calculations, the new POV being the result of a contact on the image-capture device 52.

According to one example, the stabilization system 60 is configured for tracking tools relative to a patient during computer-assisted surgery. The stabilization system 60 may operate through a processing unit; and a non-transitory computer-readable memory communicatively coupled to the processing unit and comprising computer-readable program instructions executable by the processing unit for: tracking at least one tool relative to a patient with a image-capture device at an initial orientation, detecting and quantifying a change in orientation of the image-capture device from the initial orientation, automatically returning the image-capture device to the initial orientation, and resuming the tracking of the at least one tool relative to the patient with the image-capture device.

According to one example, the stabilization system 60 is configured for tracking tools relative to a patient during computer-assisted surgery. The stabilization system 60 may operate through a processing unit; and a non-transitory computer-readable memory communicatively coupled to the processing unit and comprising computer-readable program instructions executable by the processing unit for tracking at least one tool relative to a patient with a image-capture device at an initial orientation, detecting and quantifying a change in orientation of the image-capture device from the initial orientation, and resuming the tracking of the at least one tool relative to the patient with the image-capture device taking into consideration the quantifying of the change in orientation of the image-capture device from the initial orientation.

According to one example, the stabilization system 60 includes an assembly providing at least two rotational degrees of freedom (DOF), the assembly configured to interface a navigation image-capture device to a support structure or mechanism; sensors in the assembly to quantify a change of orientation for the at least two rotational DOFs; and actuators in the assembly to modify the orientation of the assembly in the at least rotational DOFs.

In the examples provided above, the stabilization system 60 may work jointly with the CAS controller 40 to block movements of the surgical robot 20 when a variation is detected. As set out above, the CAS controller 40 may drive the surgical robot 20 in an automated mode in which the surgical robot 20 moves without assistance, or in a collaborative mode with the assistance of a user. In such arrangements, when the stabilization system 60 detects a variation in orientation of the image-capture device 52, the operation of the surgical robot 20, such as its autonomous movements or collaborative movements, may be blocked until the stabilization system 60 resumes its tracking as set about above.

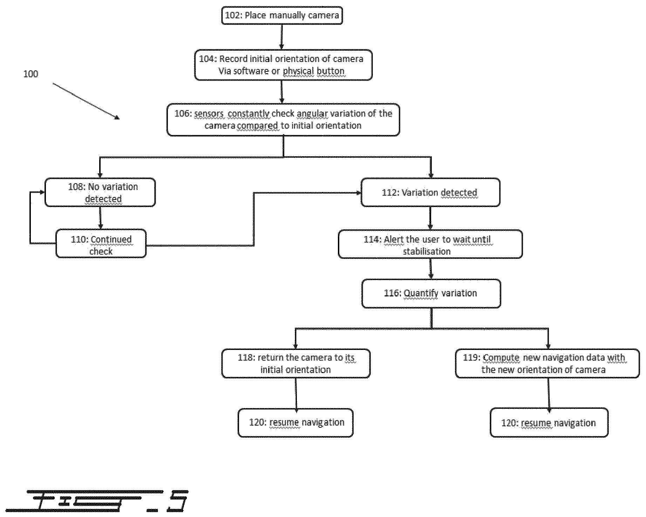

Referring to Fig. 5, an exemplary method for tracking tools relative to a patient during computer-assisted surgery is generally shown at 100. The method 100 may be performed for example using the CAS system 10, with or without the surgical robot 20, and with the tracking system 50 and stabilization system 60. The method 100 may be performed with other types of CAS systems. However, for simplicity, reference is made to components of the CAS system 10.

According to 102, the image-capture device (e.g., image-capture device 52) is positioned and/or oriented relative to the operative scene. This may for instance be done by way of manipulations of the articulated arm of links 53 and joints 54. In another embodiment, there is no 102 as the image-capture device may be adequately positioned and oriented at the start of the procedure.

According to 104, a first orientation such as the initial orientation of the image-capture device 52 is recorded, i.e., is set as being the orientation of the image-capture device 52 when tracking and is relative to the coordinate system X,Y,Z. The recordation may be triggered manually or automatically, via the software, via a button or through the interface, as possibilities. From this point on, navigation may commence, with the tracking of tool(s) and/or bone(s) using the image-capture device 52. During the tracking, as per 106, the orientation of the image-capture device is constantly verified to identify any angular variation from the initial orientation recorded in 104. By constantly, it may be included that the orientation is verified without pause, at regular time intervals, continuously, etc. This may be done by the sensors 61A-61C of the stabilization system 60, in an embodiment. This entails the continuous check 110 if no change is detected as per 108, as illustrated in Fig. 5.

According to 112, if an angular change is detected, for instance via the sensors 61A-61C of the stabilization system 60, or through inertial sensors or as declared by a user having come into contact with the image-capture device 52 or observed a contact, the method 100 may include alerting the user and/or pausing the navigation, as per 114. This may be optional, for instance if the detection of angular change is in a part of the surgical flow in which no tracking is done. Moreover, if the system may react in a negligible time lapse, or during moments where high precision is not required (e.g., the tool is at a distance from the bone), there may be no alert and/or no pause. A user may be required to validate an adjustment or quantification. If a surgical robot 20 is present, it may be blocked from moving when a change in orientation is detected, as part of pausing the navigation.

The method 100 may quantify the angular change, as per 116. This may be done using the readings from the sensors 61A-61C.

To resume the navigation with the tracking at 120, the image-capture device may be returned to its initial orientation as in 118, or the system may take into consideration the quantification of the angular change achieved in 116, in a step 119. In an embodiment featuring the stabilization system 60, the return of 118 may be performed by controlling the actuators 61A, 62A and/or 63A as a function of the quantification of 116. As another possibility, as in 119, navigation data may be calculated using the new orientation of the image-capture device 52. The quantification of 116 may indeed be used as a corrective factor to calculate tracking data from the new point of view of the image-capture device 52. Once navigation resumes, the method 100 may return to 106. Moreover, once navigation resumes, the surgical robot 20 may be allowed to move.

### Examples

The following examples can each stand on their own, or can be combined in different permutations, combinations, with one or more of other examples.

Example 1 is a method for tracking at least one tool and/or at least one bone during computer-assisted surgery, comprising: tracking at least one tool and/or at least one bone with at least one image-capture device, with the image-capture device being at a first orientation during a surgical procedure, detecting a change in orientation of the at least one image-capture device from the first orientation, quantifying the change in orientation of the at least one image-capture device from the first orientation, and tracking of the at least one tool and/or of the at least one bone with the at least one image-capture device as a function of the quantifying of the change in orientation of the image-capture device from the first orientation.

In Example 2, the subject matter of Example 1 includes, wherein tracking as a function of the quantifying of the change in orientation of the at least one image-capture device from the first orientation includes automating a return of the image-capture device to the first orientation.

In Example 3, the subject matter of Example 2 includes, wherein automating the return of the at least one image-capture device to the first orientation includes actuating a movement of the at least one image-capture device in one or two rotational degrees of freedom.

In Example 4, the subject matter of Examples 2 and 3 includes, wherein automating the return of the at least one image-capture device to the first orientation is performed automatically after the detecting and the quantifying.

In Example 5, the subject matter of Example 1 includes, wherein tracking as a function of the quantifying of the change in orientation of the image-capture device from the first orientation includes factoring in an adjustment of a point of view of the at least one image-capture device.

In Example 6, the subject matter of Examples 1 to 5 includes alerting a user of the detecting of the change in orientation of the at least one image-capture device.

In Example 7, the subject matter of Example 6, including requiring the user to quantify and/or validate the change.

In Example 8, the subject matter of Example 1 to 7 includes pausing the tracking of the at least one tool and/or at least one bone between the detecting and the qualifying, and resuming the tracking after the quantifying.

In Example 9, the subject matter of Examples 1 to 8 includes, wherein detecting the change in orientation of the at least one image-capture device from the first orientation includes detecting the change in orientation when the at least one image-capture device is coupled to a stationary structure.

In Example 10, the subject matter of Examples 1 to 9 includes, wherein detecting the change in orientation includes continuously monitoring the orientation of the at least one image-capture device.

In Example 11, the subject matter of Examples 1 to 10 includes outputting the tracking of the at least one tool and/or of the at least one bone.

In Example 12, the subject matter of Example 11 includes, wherein outputting the tracking of the at least one tool and/or of the at least one bone includes outputting the tracking graphically on a graphic-user interface.

In Example 13, the subject matter of Examples 1 to 12 includes, wherein tracking at least one tool and/or at least one bone includes tracking the at least one tool relative to the at least one bone.

In Example 14, the subject matter of Examples 1 to 13 includes, wherein tracking the at least one tool includes tracking the at least one tool as moved by a surgical robot, and further including blocking movement of the surgical robot when detecting the change in orientation.

Example 15 is a stabilization system for a navigation image-capture device in computer-assisted surgery comprising: an assembly providing at least two rotational degrees of freedom (DOF), the assembly configured to interface a navigation image-capture device to a support structure or mechanism; and at least one sensor coupled to the assembly to output data indicative of a change of orientation for the at least two rotational DOFs of the navigation image-capture device.

In Example 16, the subject matter of Example 15 includes, wherein the assembly includes a universal joint.

In Example 17, the subject matter of Examples 15 and 16 includes, wherein the at least one sensor includes at least one encoder coupled to the rotational DOFs.

In Example 18, the subject matter of Examples 16 to 17 includes at least one redundant sensor to produce data relative to the change of orientation for the at least two rotational DOFs of the navigation image-capture device.

In Example 19, the subject matter of Example 18 includes, wherein the at least one redundant sensor includes at least one inertial sensor.

In Example 20, the subject matter of Examples 15 to 19 includes a processing unit; and a non-transitory computer-readable memory communicatively coupled to the processing unit and comprising computer-readable program instructions executable by the processing unit for: tracking at least one tool and/or at least one bone with the navigation image-capture device at a first orientation during a surgical procedure, detecting the change in orientation of the navigation image-capture device from the first orientation, quantifying the change in orientation of the navigation image-capture device from the first orientation, and tracking of the at least one tool and/or of the at least one bone with the navigation image-capture device as a function of the quantifying of the change in orientation of the image-capture device from the first orientation.

In Example 21, the subject matter of Example 20 includes, wherein tracking as a function of the quantifying of the change in orientation of the navigation image-capture device from the first orientation includes factoring in an adjustment of a point of view of the navigation image-capture device.

In Example 22, the subject matter of Examples 20 and 21 includes blocking movements of a surgical robot when the change in orientation is detected.

In Example 23, the subject matter of Examples 15 to 19 includes actuators in the assembly to modify the orientation of the assembly in the at least two rotational DOFs.

In Example 24, the subject matter of Example 23, includes a processing unit; and a non-transitory computer-readable memory communicatively coupled to the processing unit and comprising computer-readable program instructions executable by the processing unit for: tracking at least one tool and/or at least one bone with the navigation image-capture device at a first orientation during a surgical procedure, detecting the change in orientation of the navigation image-capture device from the first orientation, quantifying the change in orientation of the navigation image-capture device from the first orientation, automating a return of the navigation image-capture device to the first orientation, and resuming the tracking of the at least one tool and/or of the at least one bone with the navigation image-capture device after the return of the image-capture device to the first orientation.

In Example 25, the subject matter of Example 24 includes, wherein automating the return of the navigation image-capture device to the first orientation includes actuating a movement of the at least one image-capture device in one or two of the rotational DOFs.

In Example 26, the subject matter of Examples 24 and 25 includes, wherein automating the return of the navigation image-capture device to the first orientation is performed automatically after the detecting and the quantifying.

In Example 27, the subject matter of Examples 25 or 26 includes blocking movements of a surgical robot when the change in orientation is detected and until the return of the navigation image-capture device to the first orientation.

In Example 28, the subject matter of Examples 20 to 27 includes alerting a user of the detecting of the change in orientation of the navigation image-capture device.

In Example 29, the subject matter of Example 28 includes requiring input from the user to quantify and/or validate the change.

In Example 30, the subject matter of Examples 20 to 29 includes pausing the tracking of the at least one tool and/or at least one bone between the detecting and the qualifying, and resuming the tracking after the quantifying.

In Example 31, the subject matter of Example 20 to 30 includes, wherein detecting the change in orientation of the navigation image-capture device from the first orientation includes detecting the change in orientation when the navigation image-capture device is on a stationary structure.

In Example 32, the subject matter of Examples 20 to 31 includes, wherein detecting the change in orientation includes continuously monitoring the orientation of the navigation image-capture device.

In Example 33, the subject matter of Examples 20 to 32 includes outputting the tracking of the at least one tool and/or of the at least one bone.

In Example 34, the subject matter of Example 33 includes, wherein outputting the tracking of the at least one tool and/or of the at least one bone includes outputting the tracking graphically on a graphic-user interface.

In Example 35, the subject matter of Examples 20 to 34 includes, wherein tracking at least one tool and/or at least one bone includes tracking the at least one tool relative to the at least one bone.

## Claims

1. A system for tracking at least one tool and/or at least one bone during computer-assisted surgery, comprising:
a processing unit; and
a non-transitory computer-readable memory communicatively coupled to the processing unit and comprising computer-readable program instructions executable by the processing unit for:
tracking at least one tool and/or at least one bone with at least one image-capture device, with the image-capture device being at a first orientation during a surgical procedure,
detecting a change in orientation of the at least one image-capture device from the first orientation,
quantifying the change in orientation of the at least one image-capture device from the first orientation, and
tracking of the at least one tool and/or of the at least one bone with the at least one image-capture device as a function of the quantifying of the change in orientation of the image-capture device from the first orientation.

2. The system according to claim 1, wherein tracking as a function of the quantifying of the change in orientation of the at least one image-capture device from the first orientation includes automating a return of the image-capture device to the first orientation.

3. The system according to claim 2, wherein automating the return of the at least one image-capture device to the first orientation includes actuating a movement of the at least one image-capture device in one or two rotational degrees of freedom.

4. The system according to any one of claims 2 and 3, wherein automating the return of the at least one image-capture device to the first orientation is performed automatically after the detecting and the quantifying.

5. The system according to claim 1, wherein tracking as a function of the quantifying of the change in orientation of the image-capture device from the first orientation includes factoring in an adjustment of a point of view of the at least one image-capture device.

6. The system according to any one of claims 1 to 5, including alerting a user of the detecting of the change in orientation of the at least one image-capture device.

7. The system according to claim 6, including requiring the user to quantify and/or validate the change.

8. The system according to any one of claims 1 to 7, including pausing the tracking of the at least one tool and/or at least one bone between the detecting and the qualifying, and resuming the tracking after the quantifying.

9. The system according to any one of claims 1 to 8, wherein detecting the change in orientation of the at least one image-capture device from the first orientation includes detecting the change in orientation when the at least one image-capture device is coupled to a stationary structure.

10. The system according to any one of claims 1 to 9, wherein detecting the change in orientation includes continuously monitoring the orientation of the at least one image-capture device.

11. The system according to any one of claims 1 to 10, including outputting the tracking of the at least one tool and/or of the at least one bone.

12. The system according to claim 11, wherein outputting the tracking of the at least one tool and/or of the at least one bone includes outputting the tracking graphically on a graphic-user interface.

13. The system according to any one of claims 1 to 12, wherein tracking at least one tool and/or at least one bone includes tracking the at least one tool relative to the at least one bone.

14. The system according to any one of claims 1 to 13, wherein tracking the at least one tool includes tracking the at least one tool as moved by a surgical robot, and further including blocking movement of the surgical robot when detecting the change in orientation.
